# EUROPÄISCHE PATENTSCHRIFT

(11) **EP 0 657 159 B1**
(45) Veröffentlichungstag und Bekanntmachung des Hinweises auf die Patenterteilung: **12.02.1997**
(21) Anmeldenummer: 94118382.4
(22) Anmeldetag: 23.11.1994
(51) Int. Cl.: A61K 7/13

(54) **Haarfärbemittel**
Hair dye composition
Composition tinctoriale

(30) Priorität: 09.12.1993 DE 4341998
(43) Veröffentlichungstag der Anmeldung: 14.06.1995
(73) Patentinhaber: GOLDWELL GmbH, D-64297 Darmstadt (DE)
(72) Erfinder: Lorenz, Heribert, D-64401 Gross-Bieberau (DE)

(56) Entgegenhaltungen:
- EP-A- 0 542 129
- DE-A- 4 219 981
- DE-U- 9 208 167

## Beschreibung

Die vorliegende Erfindung betrifft ein neues Haarfärbemittel für menschliches Haar, das insbesondere verbesserte Farbeigenschaften und eine ausgezeichnete toxikologische und dermatologische Verträglichkeit aufweist.

Haarfärbemittel enthalten, wie in Fachkreisen allgemein bekannt, mindestens ein Oxidationsfarbstoff-Vorprodukt, eine sogenannte Entwicklerkomponente und Kuppler, die mit Wasserstoffperoxid unmittelbar vor der Haarfärbung zusammengebracht werden und dann auf dem Haar die gewünschte Farbreaktion ergeben. Zur Feineinstellung des gewünschten Farbtones werden einer solchen Mischung üblicherweise auch Nuanceure zugesetzt.

Übliche Entwicklersubstanzen sind insbesondere aromatische Diamine wie 1,4-Diaminobenzol (p-Phenylendiamin) und 1-Methyl-2,5-diaminobenzol (p-Toluylendiamin). Diese Substanzen sind hinsichtlich ihrer dermatologischen Eigenschaften für manche Benutzer nicht optimal. Es bestand daher ein Bedürfnis, weitere Entwicklersubstanzen mit verbesserten dermatologischen und toxikologischen Eigenschaften zu finden.

In der eigenen EP-A 467 026 werden für diesen Zweck Haarfärbemittel vorgeschlagen, die als Entwicklersubstanz alleine oder in Mischung mit anderen Entwicklersubstanzen ein Hydroxytriaminopyrimidin und/oder ein Dihydroxydiaminopyrimidin enthalten, vorzugsweise 6-OH-2,4,5-triaminopyrimidin, 2-OH-4,5,6-triaminopyrimidin und 5-OH-2,4,6-triaminopyrimidin bzw. 2,6-Dihydroxy-4,5-diaminopyrimidin und/oder 4,6-Dihydroxy-2,5-diaminopyrimidin.

Als bevorzugte Kupplersubstanzen mit diesen neuen Entwicklersubstanzen werden dabei Resorcin, m-Aminophenol, m-Phenylendiamin, α-Naphthol, p-Amino-4-hydroxyethylaminoanisol, o-Aminophenol, o-Chlor-p-phenylendiamin, 1,7-Dihydroxynaphthalin und 3-Dimethylaminophenol eingesetzt, mit denen in der Regel gute Ausfärbungen erzielt werden, die jedoch nicht immer eine optimale Farbechtheit (d. h. Licht-, Wasch- und Dauerwellechtheit) aufweisen. Außerdem hat sich gezeigt, daß die Dauer der Färbung auf dem Haar für manche Farbtöne relativ kurz ist.

Die vorliegende Erfindung geht daher von der Aufgabenstellung aus, eine Haarfärbezusammensetzung zu entwickeln, die in Kombination mit Triaminohydroxypyrimidin-Entwicklern eine möglichst lang anhaltende Haarfärbung ergibt.

Die Lösung dieser Aufgabe besteht erfindungsgemäß darin, Triaminohydroxypyrimidin(e) als Entwickler enthaltenden Haarfärbemitteln zusätzlich 1,4-Diaminobenzol und/oder 1-Methyl-2,5-diaminobenzol und als Kupplersubstanz mindestens eine solche aus der Gruppe Resorcin, 2-Methylresorcin, 2-Chlorresorcin und/oder α-Naphthol alleine oder im Gemisch mit anderen Kupplern zuzusetzen.

Durch die Kombination dieser speziellen Entwicklersubstanzen mit den speziellen Kupplern werden überraschenderweise, nach Zusatz von Wasserstoffperoxid oder anderen Peroxiden, Haarfärbungen mit exzellenter Lichtechtheit und langer Haltbarkeit erreicht.

Außerdem wurde festgestellt, daß durch die Mitverwendung von 1,4-Diaminobenzol bzw. 1-Methyl-2,5-diaminobenzol in alkalisch eingestellten, Triaminohydroxypyrimidin(e) als Entwicklersubstanzen enthaltenden Färbemischungen die Reaktionsgeschwindigkeit derselben herabgesetzt werden kann, was zu verbesserten Anwendungseigenschaften führt.

Das Gewichtsverhältnis zwischen Triaminohydroxypyrimidin(en) einerseits und 1,4-Diaminobenzol bzw. 1-Methyl-2,5-diaminobenzol andererseits liegt zwischen etwa 2 : 1 und 1 : 10, vorzugsweise etwa 1 : 1 und 1 : 5, jeweils berechnet auf die freie Base.

Bevorzugte Triaminohydroxypyrimidine sind die in der bereits erwähnten EP-A 467 026 genannten Verbindungen, d. h. vorzugsweise 2,4,5-Triamino-6-hydroxypyrimidin, 4,5 6-Triamino-2-hydroxypyrimidin und 2,4,6-Triamino-5-hydroxypyrimidin. Diese Verbindungen werden, ebenso wie 1,4-Diaminobenzol und 1-Methyl-2,5-diaminobenzol, vorzugsweise in Form ihrer wasserlöslichen Salze eingesetzt, sie können jedoch selbstverständlich auch als freie Basen verwendet werden.

Der Anteil an Entwicklersubstanzen in den erfindungsgemäßen Haarfärbemitteln beträgt zwischen etwa 0,05 und 5, vorzugsweise 0,1 und 4, insbesondere 0,5 und 3 Gew.-% der Gesamtzusammensetzung (ohne Oxidationsmittel).

Obwohl an sich nicht erforderlich, können gegebenenfalls, falls zur Herstellung bestimmter Farbnuancen erwünscht, weitere an sich bekannte Entwicklersubstanzen wie 4-Aminophenol und Tetraaminopyrimidin bzw. dessen Derivate mitverwendet werden.

Die Kupplersubstanzen liegen in den erfindungsgemäßen Haarfärbemitteln etwa im gleichen Anteil wie die Entwicklersubstanzen vor, d. h. also in Mengen von 0,05 bis 5,0, vorzugsweise 0,1 bis 4, insbesondere 0,5 bis 3 Gew.-% der Gesamtzusammensetzung (ohne Oxidationsmittel).

Auch hier ist es möglich und zumeist auch zweckmäßig, weitere bekannte Kupplersubstanzen mitzuverwenden, falls dies zur Erzielung bestimmter Farbnuancen erwünscht und erforderlich ist.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten. Solche Nuanceure sind beispielsweise Nitrofarbstoffe wie 2-Amino-4,6-dinitrophenol, 2-Amino-4-nitrophenol, 2-Amino-6-chlor-4-nitrophenol etc., vorzugsweise in Mengen von etwa 0,05 bis 2,5, insbesondere 0,1 bis 1 Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K.Schrader,

"Grundlagen und Rezepturen der Kosmetika", 2. Auflage (Hüthig Buch Verlag, Heidelberg, 1989), S. 782 bis 815, beschrieben sind. Sie können als Lösungen, Cremes, Gele oder auch in Form von Aerosol-Zusammensetzungen vorliegen.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6prozentiger Konzentration.

Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, d. h. einem pH-Bereich von 5,5 bis 6,9, im neutralen als auch im alkalischen Bereich, d. h. zwischen pH 7,1 und 9,5, liegen.

Eine besonders schonende Haarfärbung wird bei Applikation einer schwach sauren Färbemittelzusammensetzung erhalten.

Die EP-A 542 129 offenbart Haarfärbemittel, die als Entwicklersubstanz mindestens ein Triaminohydroxypyrimidin und/oder Diaminohydroxypyrimidin und als Kupplersubstanz 3-Amino-2-methylamino-6-methoxypyridin enthalten.

Aus der DE-U 92 08 167.3 sind Haarfärbemittel bekannt, enthaltend eine Kombination aus einer Entwicklersubstanz, ausgewählt aus der Gruppe 1,4-Diaminobenzol, 1-Methyl-2,5-diaminobenzol, einem Tetraaminopyrimidin und einem Triaminohydroxypyrimidin, sowie einem Kupplergemisch aus 1,3-Diaminobenzol und 1,4-Diamino-2-chlorbenzol.

Im folgenden werden verschiedene Ausführungsbeispiele zur Erläuterung der Erfindung gegeben.

### Beispiele

### A

In eine Cremegrundmasse der folgenden Zusammensetzung

| | |
|---|---|
| Cetylstearylalkohol | 10,00 (Gew.-%) |
| Cocosfettsäuremonoethanolamid | 2,00 |
| Stearinsäuremonoethanolamid | 2,00 |
| Ölsäure | 2,00 |
| Natriumlaurylsulfat | 0,50 |
| Natriumsulfit | 0,40 |
| Mangandioxid | 0,12 |
| Kaliumjodid | 0,01 |
| Parfum | 0,20 |
| Wasser | ad 100,00 |

wurden jeweils, unter entsprechender Reduktion des Wassergehaltes, folgende Oxidationsfarbstoff-Vorprodukte eingearbeitet:

| Nr. 1: | |
|---|---|
| p-Toluylendiaminsulfat | 0,20 (Gew.-%) |
| 2,4,5-Triamino-6-hydroxypyrimidinsulfat | 0,20 |
| 2-Methylresorcin | 0,10 |
| 2-Aminophenol | 0,03 |
| 2-Amino-3-hydroxypyridin | 0,40 |
| HC-Red 3 | 0,20 |

| Nr. 2: | |
|---|---|
| p-Toluylendiaminsulfat | 0,25 (Gew.-%) |
| 2,4,5-Triamino-6-hydroxypyrimidinsulfat | 0,20 |
| 4-Aminophenol | 0,30 |
| 2-Methylresorcin | 0,10 |
| 4-Amino-2-methylphenol | 0,40 |
| 2-Aminophenol | 0,07 |
| Picraminsäure | 0,10 |
| HC-Red 3 | 0,25 |

| Nr. 3: | |
|---|---|
| p-Toluylendiaminsulfat | 0,29 (Gew.-%) |
| 2,4,5-Triamino-6-hydroxypyrimidinsulfat | 0,18 |
| 4-Aminophenol | 0,18 |
| 3-Aminophenol | 0,03 |
| 4-Amino-2-methylphenol | 0,40 |
| 2-Methylresorcin | 0,30 |
| Resorcin | 0,05 |
| α-Naphthol | 0,13 |
| HC-Red 3 | 0,30 |

Jeweils 1 Teil dieser Cremezusammensetzungen wurde mit 2 Teilen 2%iger H₂O₂-Lösung vermischt, wobei jeweils ein Produkt mit einem pH-Wert von 6,8 erhalten wurde, und auf menschliches Haar aufgebracht.

Nach 20- bis 25-minütiger Einwirkung wurde ausgewaschen und gespült, wobei folgende natürliche Farbtöne erhalten wurden:
- Nr. 1:: Kupfermahagoni
- Nr. 2:: Kupferblond
- Nr. 3:: Dunkelrotviolett

Diese Färbungen wiesen deutlich bessere Lichtechtheit auf und zeigten eine signifikant höhere Stabilität bei wiederholter Haarwäsche gegenüber Färbungen, die ohne die erfindungsgemäßen Kupplersubstanzen bzw. ohne Zusatz von 1-Methyl-2,5-diaminobenzol durchgeführt worden waren.

### B

In eine Cremegrundmasse der Zusammensetzung

| | |
|---|---|
| Cetylstearylalkohol | 15,00 (Gew-%) |
| Cocosfettsäuremonoethanolamid | 3,00 |
| Stearinsäuremonoethanolamid | 3,00 |
| Ölsäure | 3,00 |
| 1,2-Propandiol | 1,00 |
| Natriumlaurylsulfat | 0,50 |
| Ammoniumchlorid | 0,50 |
| Natriumsulfit | 1,00 |
| Ammoniak, 25 % | 10,00 |
| Parfum | 0,20 |
| Wasser | ad 100,00 |

wurde, unter entsprecheder Reduzierung des Wassergehaltes, eine Mischung der folgenden Oxidationsfarbstoff-Vorprodukte (in Gew.-%) eingearbeitet:

| | |
|---|---|
| p-Toluylendiaminsulfat | 0,70 |
| 2,4,5-Triamino-6-hydroxypyrimidinsulfat | 0,20 |
| 3-Aminophenol | 0,10 |
| α-Naphthol | 0,10 |
| 4-Amino-2-methylphenol | 0,18 |

1 Teil dieser Mischung wurde mit 1 Teil 6%iger wäßriger H₂O₂-Lösung gemischt, wobei sich ein pH-Wert von 9,5 einstellte, und auf das Haar aufgebracht.
Nach 30-minütiger Einwirkung wurde das Haar gewaschen und getrocknet.

Es wurde ein kräftiger, über mehrere Haarwäschen stabiler Violett-Ton erzielt.

## Patentansprüche

1. Haarfärbemittel, das als Entwicklersubstanz mindestens ein Triaminohydroxypyrimidin im Gemisch mit 1,4-Diaminobenzol und/oder 1-Methyl-2,5-diaminobenzol und zusätzlich Kupplersubstanz(en) enthält, dadurch gekennzeichnet, daß es als Kupplersubstanz(en) mindestens eine solche aus der Gruppe Resorcin, 2-Methylresorcin, 2-Chlorresorcin und/oder α-Naphthol alleine oder im Gemisch mit anderen Kupplersubstanzen enthält.

2. Haarfärbemittel nach Anspruch 1, dadurch gekennzeichnet, daß es das Gemisch aus Triaminohydroxypyrimidin und 1,4-Diaminobenzol und/oder 1-Methyl-2,5-diaminobenzol in einem Gewichtsverhältnis von 2 : 1 bis 1 : 10, berechnet auf die freie Base, enthält.

3. Haarfärbemittel nach Anspruch 2, dadurch gekennzeichnet, daß es das Gemisch aus Triaminohydroxypyrimidin und 1,4-Diaminobenzol und/oder 1-Methyl-2,5-diaminobenzol in einem Gewichtsverhältnis von 1 : 1 bis 1 : 5, berechnet auf die freie Base, enthält.

## Claims

1. Hair dyeing composition comprising as developing substance at least one triaminohydroxypyrimidine in admixture with 1,4-diaminobenzene and (or) 1-methyl-2,5-diaminobenzene and additionally coupling substance(s), characterized in that it contains as coupling substance(s) at least one compound selected from the group of resorcinol, 2-methylresorcinol, 2-chlororesorcinol and (or) α-naphthol alone or in admixture with other coupling substances.

2. Hair dyeing composition according to claim 1, characterized in that it comprises a mixture of a triaminohydroxypyrimidine and 1,4-diaminobenzene and (or) 1-methyl-2,5-diaminobenzene in a weight proportion of 2 : 1 to 1 : 10, calculated to the free bases.

3. Hair dyeing composition according to claim 2, characterized in that it contains a mixture of triaminohydroxypyrimidine and 1,4-diaminobenzene and (or) 1-methyl-2,5-diaminobenzene in a weight proportion of 1 : 1 to 1 : 5, calulated to the free bases.

## Revendications

1. Produit de teinture pour cheveux, contenant comme substance révélatrice au moins une triaminohydroxypyrimidine en mélange avec du 1,4-diaminobenzène et/ou du 1-méthyl-2,5-diaminobenzène et en outre un (des) agent(s) couplant, caractérisé en ce qu'il contient en tant qu'agent(s) couplant au moins une substance de ce type choisie parmi le résorcinol, le 2-méthylrésorcinol, le 2-chlororésorcinol et/ou l'α-naphtol, seuls ou en mélange avec d'autres agent(s) couplant.

2. Produit de teinture pour cheveux selon la revendication 1, caractérisé en ce qu'il contient le mélange de triaminohydroxypyrimidine et de 1,4-diaminobenzène et/ou de 1-méthyl-2,5-diaminobenzène en un rapport pondéral allant de 2:1 à 1:10, calculé sur la base de la base libre.

3. Produit de teinture pour cheveux selon la revendication 2, caractérisé en ce qu'il contient le mélange de triaminohydroxypyrimidine et de 1,4-diaminobenzène et/ou de 1-méthyl-2,5-diaminobenzène en un rapport pondéral allant de 1:1 à 1:5, calculé sur la base de la base libre.
